# EUROPEAN PATENT SPECIFICATION

(11) **EP 2 451 452 B1**
(45) Date of publication and mention of the grant of the patent: **07.10.2015**
(21) Application number: 10728191.7
(22) Date of filing: 23.06.2010
(51) Int. Cl.: A61K 31/416, A61P 17/06

(54) **Use of benzydamine in the treatment of p40-dependent diseases**
Verwendung von Benzydamin bei der Behandlung von p40-abhängigen Erkrankungen
Utilisation de la benzydamine dans le traitement de maladies p40-dépendantes

(30) Priority: 08.07.2009 EP 09425270
(43) Date of publication of application: 16.05.2012
(73) Proprietor: Aziende Chimiche Riunite Angelini Francesco A.C.R.A.F. S.p.A., 00181 Roma (IT)
(72) Inventor: GUGLIELMOTTI, Angelo, I-00149 Roma (IT); MANGANO, Giorgina, I-00139 Roma (IT); BIONDI, Giuseppe, I-00040 Castel Gandolfo (roma) (IT)
(74) Representative: Allaix, Roberto
(86) International application number: PCT/EP2010/058881
(87) International publication number: WO 2011/003737

(56) References cited:
- WO-A-95/03799
- WO-A-96/26724
- US-A1- 2009 155 193
- ALESSANDRINI, A.: "La benzidamina in terapia antireumatica" LA CLINICA TERAPEUTICA, vol. 39, no. 1, 15 October 1966 (1966-10-15), pages 55-64, XP009125961

## Description

### FIELD OF THE INVENTION

The present invention relates to the use of benzydamine in the treatment of p40-dependent diseases.

More in particular, the present invention relates to the use of benzydamine in the treatment of inflammatory diseases caused by an expression or overexpression of the cytokine subunit p40.

### BACKGROUND OF THE INVENTION

Benzydamine (The Merck Index, 9th ed., 1976, page 147 no.1136) was described for the first time in US 3,318,905 patent, which relates to a group of substances having analgesic, anti-inflammatory and muscle relaxant activity.

Benzydamine has been widely used in practice of human treatment as hydrochloride salt. By the systemic route it is mainly used as an antiphlogistic and analgesic. Topically it is however mainly used for those diseases which involve local inflammation such as for example myalgia, tendinitis, vulvovaginitis, gingivitis, stomatitis, mucositis of the oral cavity and so forth. Moreover benzydamine salicylate has been used in rheumatic disorders.

EP 195749 discloses the use of benzydamine in the treatment of the Trichomonas vaginalis and Gardnerella vaginalis infections. The Trichomonas vaginal is a protozoan causing infections of the genitourinary tract both in man and in woman. The Gardnerella vaginalis is a gram-variable, small, pleomorph bacillus, forming colonies of 0.25-0.44 mm diameter.

EP 812193 discloses a pharmaceutical composition which comprises an anti-inflammatory amount of benzydamine and an antimicrobially effective amount of an antimicrobial agent. The combination of benzydamine with an antimicrobial agent increases the activity of the antimicrobial agent without compromising the activity of benzydamine, so as providing an effective antiseptic, anti-inflammatory and analgesic treatment for microbial infections especially of the gums, mouth and throat.

US 5932589 discloses an oral antitussive pharmaceutical composition which allows a significant contact of its components with mucous membranes of the buccal cavity and comprises a centrally acting antitussive and benzydamine. Benzydamine was shown to be capable of shortening the onset time of the action of centrally acting antitussives on the peripheral stimulus of the cough in particular pathologies compared to the antitussive alone.

US 6300358 discloses the use of benzydamine for preparing a medicament for the treatment of pathological conditions caused by TNF, a non-glycosylated polypeptide, also known as alpha TNF or cachectin. TNF belongs to the family of cytokines and as such plays a role in the stimulation of immune responses to defend the organism from external attacks. On the other hand, an excessive action by TNF may in itself become an actual pathogenic cause given the considerable toxicity of TNF. Typical examples of the pathological conditions caused by TNF which were told to benefit of benzydamine treatment are septic shock, cachexia, chronic viral or bacterial infections such as tuberculosis or AIDS or degenerative diseases such as multiple sclerosis or ulcerative colitis.

However, the action of benzydamine is performed, in respect of TNF, at dosages higher than those administered to achieve an anti-inflammatory effect. Subsequently, doses of 20 mg/kg, and as high as 40 mg/kg in mice have been reported to be required to have a significant inhibiting effect on TNF production (M.Sironi et al., "Inhibition of inflammatory cytokine production and protection against endotoxin toxicity by benzidamine", Cytokine, Volume 8, Issue 9, September 1996, Pages 710-716 and A. Guglielmotti et al. "Benzydamine protection in a mouse model of endotoxemia", Journal Inflammation Research, Volume 46, Number 9, September, 1997, Pages 332-335).

Recently, the use of compounds capable of interfering with TNF such as etanercept, infliximab and adalimumab have been developed and clinically tested for the treatment of Crohn's disease, rheumatoid arthritis, psoriatic arthritis, psoriasis, and ankylosing spondylitis.

However, the use of compounds capable of interfering with TNF, albeit by different mechanisms, has been quite limited by safety issues. In fact, due to the key role of TNF in immune activity, clinical trials and clinical practice demonstrated that the use of anti-TNF agents is often associated with the appearance of the typical immunosuppressive sequaela such as infections, malignancy, or autoimmune disorders.

Even more recently, several scientific works indicated that cytokine subunit p40 can play a fundamental role in psoriasis pathogenesis (M. Cargill, "A large-scale genetic association study confirms IL12B and leads to the identification of IL23R as psoriasis-risk genes", American journal of human genetics, Vol. 80, Issue 2, Pg. 273-90, Feb 2007).

The cytokine subunit p40 is a component of both interleukin-12 (IL-12) and interleukin-23 (IL-23). The p40 cytokine subunit binds to the cytokine subunit p35 via a disulfide bond to form IL-12 and to cytokine subunit p19 to form IL-23. IL-12 and IL-23 are members of a small family of proinflammatory heterodimeric cytokines. The receptor for IL-12 is constituted by the subunits IL-12Rβ1 and IL-12Rβ2, while the receptor for IL-23 is constituted by the subunits IL-12Rβ1 and IL-23R.

IL-12 and IL-23 are mainly expressed by activated dendritic cells and by phagocytes. The receptors for the two cytokines are expressed on the T and NK cells, and NK T cells, but low levels of complexes of the receptor for IL-23 are also present in monocytes, macrophages and dendritic cells.

Despite these similarities, there is much evidence to suggest that IL-12 and IL-23 control different immunological circuits. Infact, whereas IL-12 controls the development of Th1 cells, which are capable of producing gamma-interferon (IFN-γ), and increases the cytotoxic, antimicrobial and antitumoral response, IL-23 regulates a circuit that leads to the generation of CD4+ cells, which are capable of producing IL-17. The induction of IL-23-dependent processes leads to the mobilization of various types of inflammatory cell, for instance TH-17, and it has been demonstrated as being crucial for the pathogenesis of numerous inflammatory pathologies mediated by immonological responses.

Typical examples of pathologies associated with the expression of p40 are chronic inflammatory diseases of the articular apparatus (e.g. rheumatoid arthritis), of the dermatological apparatus (e.g. psoriasis) and of the gastrointestinal apparatus (e.g. Crohn's disease). However, IL-23 also exerts a role in promoting tumour incidence and growth. In point of fact, IL-23 regulates a series of circuits in the tumoral microenvironment, stimulating angiogenesis and the production of inflammation mediators.

Psoriasis is a chronic inflammatory skin disease that affects 3% of the world's population (Koo J. Dermatol. Clin. 1996; 14:485-96; Schon M.P. et al., N. Engl. J. Med. 2005; 352: 1899-912). A type-1 aberrant immune response has been correlated with the pathogenesis of psoriasis, and the cytokines that induce this response, such as IL-12 and IL-23, may represent suitable therapeutic objects. The expression of IL-12 and IL-23, which share the subunit p40, is significantly increased in psoriasis plaques, and preclinical studies have demonstrated a role of these cytokines in the pathogenesis of psoriasis. More recently, the treatment of anti- IL-12 and IL-23 monoclonal antibodies of patients suffering from psoriasis proved to be effective in improving the signs of progression and seriousness of the disease and has subsequently reinforced the role of IL-12 and IL-23 in the physiopathology of psoriasis.

Crohn's disease is a chronic inflammatory pathology of the digestive apparatus and may affect any region thereof - from the mouth to the anus. It typically afflicts the terminal tract of the ileum and well-defined areas of the large intestine. It is often associated with systemic autoimmune disorders, such as mouth ulcers and rheumatic arthritis. Crohn's disease affects over 500 000 people in Europe and 600 000 people in the United States.

Crohn's disease is a pathology associated with a Th1 cell-mediated excessive activity of cytokines. IL-12 is a key cytokine in the initiation of the inflammatory response mediated by Th1 cells. Crohn's disease is characterized by increased production of IL-12 by cells presenting the antigen in intestinal tissue, and of gamma-interferon (IFN-y) and TNF-α by lymphocytes and intestinal macrophages. These cytokines induce and support the inflammatory process and thickening of the intestinal wall, which are characteristic signs of the pathology. Preclinical and clinical evidence has demonstrated that inhibition of IL-12 is effective in controlling the inflammatory response in models of intestinal inflammation and/or in patients suffering from Crohn's disease.

The relationship between cancer and inflammation is now an established fact. Many forms of tumours originate from sites of inflammation, and inflammation mediators are often produced in tumours.

IL-23 has been identified as a cytokine associated with cancer and, in particular, the expression of IL-23 is significantly high in samples of human carcinomas when compared with normal adjacent tissues. In addition, the absence of a significant expression of IL-23 in the normal adjacent tissues suggests an over-regulation of IL-23 in tumours, reinforcing its role in tumour genesis.

### SUMMARY OF THE INVENTION

The Applicant has perceived that there is still the need of developing a medicament for a safer and cheaper treatment of chronic inflammatory diseases, such as Crohn's disease, psoriatic arthritis, and psoriasis, avoiding the several adverse effects and the high costs involved with the treatments reported so far.

Now, the Applicant has surprisingly found that benzydamine is able to inhibit the expression and overexpression of the cytokine subunit p40. Moreover, the Applicant has surprisingly found that benzydamine is active on cytokine subunit p40 at lower concentrations with respect to those previously reported for its activity on TNF.

The Applicant has surprisingly found that benzydamine can be used for the treatment of inflammatory diseases caused by an expression or overexpression of the cytokine subunit p40, selected from Crohn's disease, psoriatic arthritis, and psoriasis.

Accordingly, the present invention relates to the use of benzydamine or the physiologically acceptable acid addition salts thereof in the manufacture of a medicament for the treatment of inflammatory diseases based on the expression of cytokine subunit p40 selected from Crohn's disease, psoriatric arthritis and psoriasis.

In another embodiment, the present invention relate to the use of a therapeutically effective amount of benzydamine or the physiologically acceptable acid addition salts thereof for the treatment of inflammatory diseases based on the expression of cytokine subunit p40 selected from Crohn's disease, psoriatic arthritis and psoriasis in a patient in need thereof.

Advantageously, the Applicant has found that the use of benzydamine or the physiologically acceptable acid addition salts thereof in the treatment of Crohn's disease, psoriatic arthritis, and psoriasis provides better results with respect to presently known therapeutical treatment with compounds capable of interfering with TNF.

Further, the Applicant has also found that the use of benzydamine or the physiologically acceptable acid addition salts thereof according to the present invention has lower adverse effects with respect to presently known therapeutical treatment with compounds capable of interfering with TNF.

Additionally, the Applicant has also found that the use of benzydamine or the physiologically acceptable acid addition salts thereof according to the present invention allows to provide a cheaper therapeutical treatment with respect to therapeutical treatment with compounds capable of interfering with TNF.

Last but not least, the Applicant has found that the benzydamine or the physiologically acceptable acid addition salts thereof can be used at a dosage which is lower than the dosage previously known for inhibiting the production of TNF.

Accordingly, a preferred embodiment of the present invention relates to the use of benzydamine or the physiologically acceptable acid addition salts thereof in the manufacture of a medicament for the treatment by systemic or topical administration of inflammatory diseases based on the expression of cytokine subunit p40 selected from Crohn's disease, psoriatic arthritis and psoriasis.

Advantageously, the medicament of the present invention for systemic administration comprises an amount of from 1 mg to 100 mg, more preferably from 5 mg to 50 mg, of benzydamine, expressed as free base.

Advantageously, the medicament of the present invention for topical administration comprises an amount of from 1% to 20%, more preferably from 1% to 10%, by weight of benzydamine, expressed as free base, based on the total weight of the medicament.

In a more preferred embodiment, the present invention relates to the use of benzydamine or the physiologically acceptable acid addition salts thereof at a daily dosage of from 50 mg/kg to 0.1 mg/kg of benzydamine, expressed as free base for the treatment of inflammatory diseases based on the expression of cytokine subunit p40 selected from Crohn's disease, psoriatic arthritis and psoriasis in a patient in need thereof.

Preferably, the use of the present invention comprises the systemic or topical administration.

Advantageously, the use of the present invention comprises the systemic administration of benzydamine or the physiologically acceptable acid addition salts thereof at a daily dosage of from 5 mg/kg to 0.1 mg/kg of benzydamine, expressed as free base.

Advantageously, the use of the present invention comprises the topical administration of benzydamine or the physiologically acceptable acid addition salts thereof at a daily dosage of from 50 mg/kg to 1 mg/kg of benzydamine, expressed as free base.

### DETAILED DESCRIPTION OF THE INVENTION

For the purpose of the present invention, benzydamine or the physiologically acceptable acid addition salts thereof may be administered as such.

Advantageously, benzydamine or the physiologically acceptable acid addition salts thereof may be administered in the form of pharmaceutical preparations for systemic or topical administration.

Benzydamine can form physiologically acceptable acid addition salts with physiologically acceptable organic or mineral acids. Typical examples of physiologically acceptable mineral acids are hydrochloric acid, hydrobromic acid, sulfuric acid, phosphoric acid and nitric acid. Typical examples of suitable physiologically acceptable organic acids are acetic acid, ascorbic acid, benzoic acid, citric acid, fumaric acid, lactic acid, maleic acid, methanesulfonic acid, oxalic acid, para-toluenesulfonic acid, benzensulfonic acid, succinic acid, tannic acid and tartaric acid.

When used by systemic route, the pharmaceutical preparations may be solid, like tablets, dragees, capsules, powders and slow-release forms or liquid, such as sterile solutions for intramuscular or intravenous injections, suspensions and emulsions.

The pharmaceutical preparations for topical use may be vaginal dosage forms like solutions for lavages, creams and foams, dosage forms for treating the buccal cavity like mouth washings and sprays, as well as dosage forms for the nose and the ear such as ointments, pastes, creams, foams, gels, solutions and powders.

In addition to conventional excipients, the preparations of the present invention may comprise other suitable pharmaceutical additives such as preservatives, stabilisers, emulsifiers, salts for regulating osmotic pressure, buffers, colouring and flavouring agents.

When required by particular therapies, the preparations of the present invention may also comprise other compatible active ingredients whose simultaneous administration is helpful.

Advantageously, the pharmaceutical preparations of the present invention for systemic administration comprise an amount of from 1 mg to 100 mg, more preferably from 5 mg to 50 mg, of benzydamine, expressed as free base.

Advantageously, the pharmaceutical preparations of the present invention for topical administration comprises an amount of from 1% to 20%, more preferably from 1% to 10%, by weight of benzydamine, expressed as free base, based on the total weight of the medicament.

For practical uses in therapy the effective amount of benzydamine or the physiologically acceptable acid addition salts thereof may vary over a rather broad range depending on known factors, such as the specific treatment required, the selected pharmaceutical preparation and the administration route, as well as the age, weight and response of the particular patient. However, the optimum effective amount and interval of dosing can easily be accomplished by the physician concerned according to simple routine procedures.

In general, the daily dosage will preferably be lower than 50 mg/kg, more preferably lower than 10 mg/kg, and even more preferably lower than 5 mg/kg of benzydamine free base.

On the other hand, the daily dosage will preferably be higher than 0.1 mg/kg, preferably higher than 0,5 mg/kg, and even more preferably higher than 1 mg/kg of benzydamine free base.

Of course, in the case of an acid addition salt thereof, an amount corresponding to the abovementioned amount of benzydamine free base will be administered.

Advantageously, in case of systemic administration, the daily dosage will preferably be from 5 mg/kg to 0.1 mg/kg of benzydamine, expressed as free base.

Advantageously, in case of topical administration, the daily dosage will preferably be from 50 mg/kg to 1 mg/kg of benzydamine, expressed as free base.

The pharmaceutical preparations of the present invention can be made following the conventional techniques of the pharmaceutical chemist involving mixing, granulating and compressing when necessary, or variously mixing and dissolving the ingredients as appropriate to give the desired result.

The following examples will illustrate at least one way of carrying out the invention, without however in any way restricting the matter for which protection is sought which is defined by the annexed claims.

### EXAMPLE 1

### Effect of benzvdamine on p40 mRNA expression in vitro.

The capacity of benzydamine to inhibit the expression of p40 by lipopolysaccharide (LPS)-stimulated MonoMac6 cells was evaluated.

The cells were placed in 96-well plates at a concentration of 50 000 cells/well. The compound was tested at the concentration of 75µM and incubated for 1 hour. The cells were then stimulated with LPS (100 ng/ml) for 4 hours.

The total RNA was extracted from the cell pellet using the RNeasy mini kit (Qiagen), reverse-transcribed with the TaqMan Reverse transcription reagents synthesis kit (Applied Biosystems) and the cDNA obtained was used for the real-time PCR reaction.

The amplification was obtained in 96-well plates using the ABI Prism 7000 sequence detection system (Applied Biosystems), by applying the following temperature profile: 50°C for 2 minutes, 95°C for 10 minutes and 45 cycles at 95°C for 15 seconds and 60°C for 1 minute. For the amplification, a set of primers and probe specific for human p40 was used (Applied Biosystems, RefSeq NM_002187.2).

A set of primers and probe for β-actin was used in separate wells as an internal control of the samples for the purposes of normalization. Once the reaction had taken place, the fluorescence data were analysed using the ABI Prism 7000 SDS software, by calculating the threshold cycle (Ct) for each sample and subsequently performing a relative quantification via the ΔΔCt method.

The results obtained, expressed as a percentage of inhibition, are collated in Table 1 below.

**TABLE 1**

| Benzydamine [µM] | % inhibition |
|---|---|
| 75 | 80 |

As shown by the results obtained and given in Table 1, benzydamine was capable of significantly inhibiting the LPS-induced expression of p40 in a human monocyte line, showing 80% reduction in the levels of specific mRNA.

### EXAMPLE 2

### Effect of benzydamine on p40 protein production in vitro.

The capacity of the compound to inhibit the production of the protein p40 by anti-CD3 stimulated human PBMCs (peripheral blood mononuclear cells) was evaluated.

The cells were placed in 96-well plates at a concentration of 2x10⁵ cells/well. The compound was tested on a 6-point ½-log dose curve (in the range 0.3-100 µM) and incubated for 1 hour. The cells were then stimulated with anti-CD3 (4 µg/ml) for 48 hours.

The amount of p40 produced was measured in the supernatants, suitably diluted with buffer, by using the Luminex 100 system.

The results obtained are shown in Table 2 below.

**TABLE 2**

| Benzydamine [µM] | % inhibition |
|---|---|
| 100 | 99 |
| 30 | 97 |
| 10 | 44 |
| 3 | 24 |
| 1 | 23 |
| 0 | 0 |

As shown by the results obtained and given in Table 1, the compound was capable of significantly inhibiting the anti-CD3 induced production of p40 in human PBMCs, showing an inhibition in the range 23-99% and resulting in an IC50 of 10.1µM.

### EXAMPLE 3

### Effect of benzvdamine on TNF in vitro.

The capacity of the compound to inhibit the expression of the protein TNF by anti-CD3 stimulated human PBMCs (peripheral blood mononuclear cells) was evaluated.

The cells were placed in 96-well plates at a concentration of 2x10⁵ cells/well. The compound was tested on a 6-point ½-log dose curve (in the range 0.3-100 µM) and incubated for 1 hour. The cells were then stimulated with anti-CD3 (4 µg/ml) for 48 hours.

The amount of TNF produced was measured in the supernatants, suitably diluted with buffer, by using the Luminex 100 system.

The results obtained are reported in Table 3 below.

**TABLE 3**

| Benzydamine [µM] | % inhibition |
|---|---|
| 100 | 70 |
| 30 | 44 |
| 10 | 22 |
| 3 | 12 |
| 1 | 8 |
| 0 | 7 |

As shown by the results obtained and given in Table 3, the compound was capable of inhibiting the anti-CD3 induced production of TNF in human PBMCs in the range 8-70%, resulting in an IC50 of 43.1µM that is about 4 fold higher than that obtained with p40.

### EXAMPLE 4

### Effect of benzvdamine on plaque psoriasis

Given the lack of reliable experimental animal models for psoriasis (Lowes MA et al. Nature 2007; 445:866-73) and taking into account the long lasting experience with benzydamine in human, its low systemic exposure after topical administration and the excellent safety profile, benzydamine was tested as a 5% cream (corresponding to the formulation of the following table 8) in a small group of patients affected by psoriasis.

The cream was tested in seven patients affected by mild to moderate forms of plaque psoriasis (PASI≤10). PASI (Psoriasis Area Severity Index) is a validated measure of average severity of the skin lesions, assessed as "redness", "thickness", and "scaling", weighted for percent of area involved and location of the lesions. The cream was applied, massaged lightly into the affected area until it is absorbed by the skin, once a day for 30 consecutive days.

Treatment with benzydamine 5% cream resulted effective in 4 out of the 7 treated patients showing a significant ≥50% reduction in PASI.

### EXAMPLE 5

The following tables 4 to 9 show specific examples of pharmaceutical preparations according to the present invention.

**TABLE 4**

| Benzydamine 50 mg coated tablet | |
|---|---|
| | Amount (mg) |
| Active Substance | |
| Benzydamine Hydrochloride | 50 |

| Excipients | |
|---|---|
| Starch | 18.95 |
| Microcrystalline cellulose | 65.25 |
| Dibasic calcium phosphate | 28 |
| Talc | 5 |
| Magnesium stearate | 1 |
| Colloidal silicon dioxide | 1.8 |
| Hypromellose | 1.5 |
| PEG 6000 | 2.85 |
| Acacia | 1.5 |
| Titanium dioxide | 0.8 |
| Sucrose | 103 |
| White wax | 0.105 |
| Carnauba wax | 0.015 |

**TABLE 5**

| Benzydamine 3% oral drops | |
|---|---|
| | Amount (g) |
| Active Substance | |
| Benzydamine Hydrochloride | 3 |

| Excipients | |
|---|---|
| Metyl p-idrossibenzoate | 0.18 |
| Propyl p-idrossibenzoate | 0.02 |
| Saccharin | 0.258 |
| Sodium bicarbonate | 0.118 |
| Mint flavour | 0.5 |
| Purified water q.s. to 100 ml | |

**TABLE 6**

| Benzydamine 25mg ampuls | |
|---|---|
| | Amount (mg) |
| Active Substance | |
| Benzydamine Hydrochloride | 25 |
| Excipients | |
| Sterile water for injection q.s. to 5 ml | |

**TABLE 7**

| Benzydamine 3% cream | |
|---|---|
| | Amount (g) |
| Active Substance | |
| Benzydamine Hydrochloride | 3 |

| Excipients | |
|---|---|
| White Soft Paraffin | 15 |
| Cetyl alcohol | 10 |
| Propylene glycol | 10 |
| Polysorbate 60 | 3 |
| Sorbitan stearate 60 | 3 |
| Purified water q.s. to 100 g | |

**TABLE 8**

| Benzydamine 5% cream | |
|---|---|
| | Amount (g) |
| Active Substance | |
| Benzydamine Hydrochloride | 5 |

| Excipients | |
|---|---|
| Glyceryl and polyoxyethylenglycol palmitostearate | 18 |
| Decyl oleate | 5 |
| Propylene glycol | 5 |
| Lauroyl macrogolglycerides | 3 |
| Fluid silicone | 1 |
| Perfume | 0.2 |
| Methyl parahydroxybenzoate | 0.18 |
| Propyl parahydroxybenzoate | 0.02 |
| Purified water q.s. to 100g | |

**TABLE 9**

| Benzydamine 5% gel | |
|---|---|
| | Amount (g) |
| Active Substance | |
| Benzydamine Hydrochloride | 5 |

| Excipients | |
|---|---|
| Isopropyl alcohol | 20 |
| Glycerol | 2 |
| Hydroxyethylcellulose | 1.8 |
| Perfume | 0.2 |
| Purified water q.s. to 100g | |

## Claims

1. The use of benzydamine or the physiologically acceptable acid addition salts thereof in the manufacture of a medicament for the treatment of an inflammatory disease caused by the expression of cytokine subunit p40, wherein said inflammatory disease is selected from the group consisting of Crohn's disease, psoriatic arthritis, and psoriasis.

2. The use according to claim 1, wherein said medicament is formulated for systemic or topical administration.

3. The use according to claim 2, wherein said medicament for systemic administration comprises an amount of from 1 mg to 100 mg, more preferably from 5 mg to 50 mg, of benzydamine, or the physiologically acceptable acid addition salts thereof, expressed as free base.

4. The use according to claim 2, wherein said medicament for topical administration comprises an amount of from 1% to 20%, more preferably from 1% to 10%, by weight of benzydamine, or the physiologically acceptable acid addition salts thereof, expressed as free base, based on the total weight of the medicament.

5. The use according to claim 2, wherein said medicament is administered at a daily dosage of from 50 mg/kg to 0.1 mg/kg of benzydamine, or the physiologically acceptable acid addition salts thereof, expressed as free base.

6. The use according to claim 2, wherein said medicament for systemic administration is administered at a daily dosage of from 5 mg/kg to 0.1 mg/kg of benzydamine, or the physiologically acceptable acid addition salts thereof, expressed as free base.

7. The use according to claim 2, wherein said medicament for topical administration is administered at a daily dosage of from 50 mg/kg to 1 mg/kg of benzydamine, or the physiologically acceptable acid addition salts thereof, expressed as free base.

8. A pharmaceutical composition for use in the treatment of an inflammatory disease caused by the expression of cytokine subunit p40 by systemic or topical administration, wherein said inflammatory disease is selected from the group consisting of Crohn's disease, psoriatic arthritis, and psoriasis wherein said pharmaceutical composition comprises benzydamine or the physiologically acceptable acid addition salts thereof.

9. The pharmaceutical composition according to the use of claim 8, wherein said pharmaceutical composition comprises an amount of from 1 mg to 100 mg, more preferably from 5 mg to 50 mg, of benzydamine, or the physiologically acceptable acid addition salts thereof, expressed as free base, wherein the composition is for systemic administration.

10. The pharmaceutical composition according to the use of claim 8, wherein said pharmaceutical composition comprises of from 1% to 20%, more preferably from 1% to 10%, by weight of benzydamine, or the physiologically acceptable acid addition salts thereof, expressed as free base, based on the total weight of the composition, wherein the composition is for topical administration.

11. The pharmaceutical composition according to the use of any one of claims 8 to 10, wherein said physiologically acceptable acid addition salt of benzydamine is obtained with a mineral or organic acid selected from the group comprising hydrochloric acid, hydrobromic acid, sulfuric acid, phosphoric acid, nitric acid, acetic acid, ascorbic acid, benzoic acid, citric acid, fumaric acid, lactic acid, maleic acid, methanesulfonic acid, oxalic acid, para-toluenesulfonic acid, benzensulfonic acid, succinic acid, tannic acid and tartaric acid, wherein the composition is for systemic or topical administration.

## Patentansprüche

1. Verwendung von Benzydamin oder physiologisch annehmbaren Säureadditionssalzen davon in der Herstellung eines Medikaments zur Behandlung einer Entzündungskrankheit, die durch Expression der Zytokin-Untereinheit p40 ausgelöst wird, wobei die Entzündungskrankheit ausgewählt ist unter Morbus Crohn, Psoriasisarthritis und Psoriasis.

2. Verwendung nach Anspruch 1, wobei das Medikament für die systemische oder topische Verabreichung formuliert ist.

3. Verwendung nach Anspruch 2, wobei das Medikament für die systemische Verabreichung eine Menge von 1 mg bis 100 mg, stärker bevorzugt 5 mg bis 50 mg, Benzydamin oder physiologisch annehmbare Säureadditionssalze davon, berechnet als freie Base, umfasst.

4. Verwendung nach Anspruch 2, wobei das Medikament für die topische Verabreichung eine Menge von 1 Gew.-% bis 20 Gew.-%, stärker bevorzugt 1 Gew.-% bis 10 Gew.-%, Benzydamin oder physiologisch annehmbare Säureadditionssalze davon, berechnet als freie Base, bezogen auf das Gesamtgewicht des Medikaments, umfasst.

5. Verwendung nach Anspruch 2, wobei das Medikament mit einer Tagesdosis von 50 mg/kg bis 0,1 mg/kg Benzydamin oder physiologisch annehmbaren Säureadditionssalzen davon, berechnet als freie Base, verabreicht wird.

6. Verwendung nach Anspruch 2, wobei das Medikament für die systemische Verabreichung mit einer Tagesdosis von 5 mg/kg bis 0,1 mg/kg Benzydamin oder den physiologisch annehmbaren Säureadditionssalzen davon, ausgedrückt als freie Base, verabreicht wird.

7. Verwendung nach Anspruch 2, wobei das Medikament für die topische Verabreichung mit einer Tagesdosis von 50 mg/kg bis 1 mg/kg an Benzydamin oder physiologisch annehmbaren Säureadditionssalzen davon, berechnet als freie Base, verabreicht wird.

8. Pharmazeutische Zusammensetzung zur Verwendung in der Behandlung einer Entzündungskrankheit, die durch die Expression der Zytokin-Untereinheit p40 ausgelöst wird, durch systemische oder topische Verabreichung, wobei die Entzündungskrankheit ausgewählt ist unter Morbus Crohn, Psoriasisarthritis und Psoriasis, wobei die pharmazeutische Zusammensetzung Benzydamin oder physiologisch annehmbare Säureadditionssalze davon umfasst.

9. Pharmazeutische Zusammensetzung zur Verwendung nach Anspruch 8, wobei die pharmazeutische Zusammensetzung eine Menge von 1 mg bis 100 mg, stärker bevorzugt von 5 mg bis 50 mg, Benzydamin oder physiologisch annehmbaren Säureadditionssalze davon, berechnet als freie Base, umfasst, wobei die Zusammensetzung für die systemische Verabreichung angepasst ist.

10. Pharmazeutische Zusammensetzung zur Verwendung nach Anspruch 8, wobei die pharmazeutische Zusammensetzung 1 Gew.-% bis 20 Gew.%, stärker bevorzugt 1 Gew.-% bis 10 Gew.-%, Benzydamin oder physiologisch annehmbare Säureadditionssalze davon, berechnet als freie Base, bezogen auf das Gesamtgewicht der Zusammensetzung, umfasst, wobei die Zusammensetzung für die topische Verabreichung angepasst ist.

11. Pharmazeutische Zusammensetzung zur Verwendung nach einem der Ansprüche 8 bis 10, wobei das physiologisch annehmbare Säureadditionssalz von Benzydamin mit einer Mineralsäure oder organischen Säure, ausgewählt unter Chlorwasserstoffsäure, Bromwasserstoffsäure, Schwefelsäure, Phosphorsäure, Salpetersäure, Essigsäure, Ascorbinsäure, Benzoesäure, Zitronensäure, Fumarsäure, Milchsäure, Maleinsäure, iVlethansulfonsäure, Oxalsäure, para-Toluolsulfonsäure, Benzolsulfonsäure, Bernsteinsäure, Gerbsäure und Weinsäure erhalten ist, wobei die Zusammensetzung für die systemische oder topische Verabreichung angepasst ist.

## Revendications

1. Utilisation de benzydamine ou de ses sels d'addition d'acide physiologiquement acceptables dans la fabrication d'un médicament pour le traitement d'une maladie inflammatoire provoquée par l'expression de la sous-unité de cytokine p40, dans laquelle ladite maladie inflammatoire est choisie dans le groupe constitué par la maladie de Crohn, le rhumatisme psoriasique, et le psoriasis.

2. Utilisation selon la revendication 1, dans laquelle ledit médicament est formulé pour une administration par voie systémique ou topique.

3. Utilisation selon la revendication 2, dans laquelle ledit médicament pour administration par voie systémique comprend une quantité de 1 mg à 100 mg, mieux encore de 5 mg à 50 mg, de benzydamine, ou de ses sels d'addition d'acide physiologiquement acceptables, exprimée sous forme de base libre.

4. Utilisation selon la revendication 2, dans laquelle ledit médicament pour administration par voie topique comprend une quantité de 1 % à 20 %, mieux encore de 1 % à 10 % en poids de benzydamine, ou de ses sels d'addition d'acide physiologiquement acceptables, exprimée sous forme de base libre, par rapport au poids total du médicament.

5. Utilisation selon la revendication 2, dans laquelle ledit médicament est administré à une dose quotidienne de 50 mg/kg à 0,1 mg/kg de benzydamine, ou de ses sels d'addition d'acide physiologiquement acceptables, exprimée sous forme de base libre.

6. Utilisation selon la revendication 2, dans laquelle ledit médicament pour administration par voie systémique est administré à une dose quotidienne de 5 mg/kg à 0,1 mg/kg de benzydamine, ou de ses sels d'addition d'acide physiologiquement acceptables, exprimée sous forme de base libre.

7. Utilisation selon la revendication 2, dans laquelle ledit médicament pour administration par voie topique est administré à une dose quotidienne de 50 mg/kg à 1 mg/kg de benzydamine, ou de ses sels d'addition d'acide physiologiquement acceptables, exprimée sous forme de base libre.

8. Composition pharmaceutique pour utilisation dans le traitement d'une maladie inflammatoire provoquée par l'expression de la sous-unité de cytokine p40 par administration systémique ou topique, dans laquelle ladite maladie inflammatoire est choisie dans le groupe constitué par la maladie de Crohn, le rhumatisme psoriasique, et le psoriasis, laquelle composition pharmaceutique comprend de la benzydamine ou ses sels d'addition d'acide physiologiquement acceptables.

9. Composition pharmaceutique selon l'utilisation de la revendication 8, laquelle composition pharmaceutique comprend une quantité de 1 mg à 100 mg, mieux encore de 5 mg à 50 mg, de benzydamine, ou de ses sels d'addition d'acide physiologiquement acceptables, exprimée sous forme de base libre, et laquelle composition est destinée à être administrée par voie systémique.

10. Composition pharmaceutique selon l'utilisation de la revendication 8, laquelle composition pharmaceutique comprend de 1 % à 20 %, mieux encore de 1 % à 10 % en poids de benzydamine, ou de ses sels d'addition d'acide physiologiquement acceptables, exprimés sous forme de base libre, et laquelle composition est destinée à être administrée par voie topique.

11. Composition pharmaceutique selon l'utilisation de l'une quelconque des revendications 8 à 10, dans laquelle ledit sel d'addition d'acide physiologiquement acceptable de benzydamine est obtenu avec un acide minéral ou organique choisi dans le groupe comprenant l'acide chlorhydrique, l'acide bromhydrique, l'acide sulfurique, l'acide phosphorique, l'acide nitrique, l'acide acétique, l'acide ascorbique, l'acide benzoïque, l'acide citrique, l'acide fumarique, l'acide lactique, l'acide maléique, l'acide méthanesulfonique, l'acide oxalique, l'acide paratoluènesulfonique, l'acide benzènesulfonique, l'acide succinique, l'acide tannique et l'acide tartrique, laquelle composition est destinée à être administrée par voie systémique ou topique.
